# EUROPÄISCHE PATENTSCHRIFT

(11) **EP 3 978 037 B1**
(45) Veröffentlichungstag und Bekanntmachung des Hinweises auf die Patenterteilung: **13.09.2023**
(21) Anmeldenummer: 20199176.7
(22) Anmeldetag: 30.09.2020
(51) Int. Cl.: A61L 9/12, C04B 33/04, C04B 33/32

(54) **SYSTEM ZUM BEDUFTEN VON RÄUMEN**
SYSTEM FOR ODORIZING AREAS
SYSTÈME DE DÉSODORISATION DE PIÈCES

(43) Veröffentlichungstag der Anmeldung: 06.04.2022
(73) Patentinhaber: Stearinos OOD, 7500 Silistra (BG)
(72) Erfinder: WITTEKIND, Herbert, 7500 Silistra (BG)
(74) Vertreter: WSL Patentanwälte Partnerschaft mbB

(56) Entgegenhaltungen:
- JP-A- 2008 081 330
- US-A1- 2019 098 935
- DELE-AFOLABI T T ET AL: "Research trend in the development of macroporous ceramic components by pore forming additives from natural organic matters: A short review", CERAMICS INTERNATIONAL, ELSEVIER, AMSTERDAM, NL, Bd. 43, Nr. 2, 29. Oktober 2016 (2016-10-29), Seiten 1633-1649, XP029843137, ISSN: 0272-8842, DOI: 10.1016/J.CERAMINT.2016.10.177

## Beschreibung

Die vorliegende Erfindung betrifft ein System zum Beduften von Räumen. Das System umfasst einen Körper, der eine flüssige Mischung mit wenigstens einem Duftstoff aufnimmt, sowie die Mischung. Ferner wird ein Verfahren zur Herstellung eines solchen Körpers ein Verfahren zur Aufbereitung des Systems sowie ein Mischungs-Spender beschrieben.

Bereits seit langem ist es üblich, der Luft Geruchsstoffe (Riechstoffe, Duftstoffe) zuzusetzen. Das gezielte Beduften von Räumen dient dazu, den sich in dem Raum befindenden Personen den Aufenthalt in dem jeweiligen Raum angenehmer zu machen, weil etwaige unangenehme Gerüche kaschiert werden oder weil mittels der Duftstoffe ein bestimmtes positives Gefühl (z.B. einen Wiedererkennungseffekt) ausgelöst werden kann, beispielsweise in Hotels oder Supermärkten. Die Verwendung von Duftstoffen basiert auf der Beobachtung, dass für den Menschen das Riechen neben dem Sehen, Hören oder Tasten ein wichtiger Sinneseindruck darstellt. Hierbei ist der Geruch (d.h. die olfaktorische Wahrnehmung) die Interpretation von Sinneserregungen, die von den Chemorezeptoren der Nase oder anderer Geruchsorgane an das Gehirn übermittelt werden. Einer aktuellen Studie zufolge soll der Mensch über eine Billion Gerüche unterscheiden können.

Für die Einbringung von Duftstoffen in die Luft sind verschiedene Systeme bekannt. Eine einfache und weit verbreitete Möglichkeit stellt die Verwendung von Duftkerzen dar. Duftkerzen brennen jedoch schnell ab und sind für eine dauerhafte Verwendung nicht geeignet. Durch die Flamme besteht zudem Brandgefahr.

Das Dokument US 2019/0098935 A1 zeigt und beschreibt einen gesinterten Körper mit einer elektrisch leitenden Beschichtung und einer Porosität von 10 bis 90 %. Der gesinterte Körper wird als Verdampfer insbesondere für elektronische Zigaretten verwendet. Die elektrisch leitende Beschichtung, an die Spannung angelegt werden kann, dient zum Erwärmen des Körpers.

Aus dem Dokument JP 2008 081330 A ist ein poröser keramischer Körper für die Aufnahme einer Flüssigkeit bekannt, der eine tri-modale Porengrößenverteilung aufweist.

Aus der Druckschrift EP 3 488 872 A1 ist ein Duftstoffspender mit einem Duftstoffreservoir bekannt, welches in einem Gehäuse aufgenommen ist, wobei das Gehäuse als Unterputzgehäuse ausgebildet ist. Der Duftstoff kann in einem Duftstoffreservoir aus einem porösen, luftdurchlässigen Formkörper aus einer Vielzahl von Kunststoffpartikeln aufgenommen sein, wobei die Kunststoffpartikel Duftstoffe umfassen. Wenn ein solcher Duftstoffspender über einen langen Zeitraum genutzt werden soll, muss das Duftstoffreservoir (d.h. der Formkörper mit den Duftstoff-Kunststoffpartikeln) nach einer gewissen Zeit durch ein neues Duftstoffreservoir ersetzt werden. Das verbrauchte Duftstoffreservoir wird entsorgt.

Für das Beduften von Räumen werden derzeit häufig Sprühsysteme mit Spraydosen verwendet, welche in regelmäßigen Zeitabständen einen Sprühstoß mit Duftstoff absetzen. Es werden beispielsweise Spraydosen mit einem Fassungsvermögen von 100 ml bis 150 ml eingesetzt, welche einen Raum ca. 4 bis 6 Wochen beduften können. Anschließend wird die leere Spraydose entsorgt. Dies erzeugt viel Müll.

Derzeit und in der Zukunft gewinnt der Umweltschutz immer stärker an Bedeutung. Vor diesem Hintergrund wurde nach Lösungen gesucht, wie ein System zum Beduften von Räumen ressourcenschonender gestaltet werden kann. Zugleich sollte ein System zum Beduften von Räumen geschaffen werden, das eine lange Wirkungszeit aufweist, um den Aufwand bei der Benutzung eines solchen Systems zu minimieren. Zugleich sollte die Möglichkeit bestehen, Düfte zu wechseln, um durch die Verwendung eines neuen Duftes nach einer bestimmten Zeit zu ermöglichen, da ein Duftstoff nach einer gewissen Zeit gar nicht mehr oder in geringerer Stärke wahrgenommen wird.

Die obige Aufgabe wird gelöst durch ein System zum Beduften von Räumen mit den Merkmalen des Anspruchs 1 sowie ein Verfahren zur Aufbereitung des Systems mit den Merkmalen des Anspruchs 7.

Die obige Aufgabe wird insbesondere gelöst durch ein System zum Beduften von Räumen mit einem eine flüssige Mischung aufnehmenden, festen Körper und der flüssigen Mischung, welche mindestens einen Duftstoff enthält, wobei der Körper ein poröses, keramisches und wiederverwendbares Material aufweist, das eine offene Porosität besitzt, die größer als 50 % ist. Erfindungsgemäß weist die Mischung einen Flammpunkt auf, der gleich oder kleiner als 120 °C ist.

Das erfindungsgemäße System setzt sich zusammen aus dem festen, selbsttragenden, keramischen, porösen Körper, der zur Aufnahme und zum Verdunsten der zunächst flüssigen Mischung dient, und aus der Mischung selbst. Die Mischung mit ihrem wenigstens einen Duftstoff zum Beduften des Raumes geht durch Verdunstung nach und nach in den gasförmigen Zustand über und entweicht über einen vorgegebenen Zeitraum aus dem porösen Körper. Die offene Porosität, die größer als 50 % ist, vorzugsweise größer als 80 %, besonders bevorzugt größer als 90 % ist, ermöglicht die leichte Aufnahme bzw. die gewünscht leichte Abgabe der Mischung über Verdunsten in den bzw. aus dem Körper. Hierbei wird als offene Porosität der Anteil der Hohlräume des Körpers bezeichnet, der untereinander bzw. mit der Umgebung in Verbindung ist. Demgegenüber bilden Hohlräume eine geschlossene Porosität, wenn diese gegeneinander bzw. gegen die Umgebung abgeschlossen sind. Hierbei beziehen sich die obigen Angaben auf das Volumen der Poren, nämlich das Volumen der offenen Poren in Bezug auf das Volumen aller Poren (offene Porosität) bzw. das Volumen der geschlossenen Poren in Bezug auf das Volumen aller Poren. Die Porosität kann beispielsweise mittels Quecksilber-Porosimetrie bestimmt werden.

Zudem weist das keramische Material eine bimodale Porengrößenverteilung auf, wenn die Häufigkeit des Vorkommens von Poren hinsichtlich ihrer Größe aufgetragen wird. Als Porengröße wird hierbei der innere Durchmesser des Hohlraums (Pore) verstanden. Dies bedeutet, dass sich die meisten Poren des keramischen Materials einer ersten Gruppe mit einem größeren Porendurchmesser oder einer zweiten Gruppe mit einem kleineren Porendurchmesser zuordnen lassen. Diese Porengrößenverteilung bewirkt, dass die Abgabegeschwindigkeit der Mischung in dem gewünschten Bereich liegt. Beispielsweise können 50 g Mischung einen Raum einer Größe von 20 m² über einen Zeitraum im Bereich von 1 Woche bis 6 Monate beduften. Zudem wird die Mischung vollständig in den Körper aufgenommen, so dass die Mischung bei Berührung des Körpers nicht austritt.

Die Porengröße der ersten Gruppe von Poren (d.h. ein erstes Maximum der bimodalen Porengrößenverteilung) liegt im Bereich zwischen 1 mm und 3 mm, vorzugsweise zwischen 2 mm und 3 mm, und die Porengröße der zweiten Gruppe von Poren (d.h. ein zweites Maximum der Porengrößenverteilung) im Bereich zwischen 0,01 mm und 0,3 mm, vorzugsweise zwischen 0,05 mm und 0,3 mm. Die erfindungsgemäße Kombination von großen und kleinen Poren führt (zusammen mit der hierfür angepassten, unten beschriebene Mischung) dazu, dass die Aufnahmekapazität des Körpers für die wenigstens einen Duftstoff enthaltende Flüssigkeit sehr hoch ist. Um eine Vergleichbarkeit der Körper bei der Verwendung unterschiedlicher Substanzen zu erzielen, wird die Aufnahmekapazität des Körpers mittels Wasser gemessen. Bei einem erfindungsgemäßen Körper beträgt die Wasseraufnahme mindestens 70 % des Gewichts des Körpers, vorzugsweise mindestens 80 % des Gewichts des Körpers, besonders bevorzugt mindestens 90 % des Gewichts des Körpers. Dies bedeutet beispielsweise, dass, wenn der Körper ein Gewicht von 300 g hat, die Aufnahmekapazität an Wasser mindestens 210 g, vorzugsweise mindestens 240 g, besonders bevorzugt mindestens 270 g beträgt. Die hohe Aufnahmekapazität des porösen Körpers bewirkt, dass eine lange Beduftungszeit realisiert werden kann, ohne dass Wartungs- und Nachfüllarbeiten erforderlich sind.

Alternativ (nicht zur Erfindung gehörend) kann der Körper überwiegend Poren mit einem kleinen Porendurchmesser aufweisen, wobei der Median der Porengrößenverteilung in einem Bereich zwischen 0,01 mm und 0,3 mm liegt, vorzugsweise zwischen 0,05 mm und 0,3 mm. Bei dieser Porengröße ist die Aufnahmekapazität des Körpers für die Mischung ebenfalls noch hoch, jedoch insgesamt etwas geringer als bei dem Körper mit der bimodalen Porengrößenverteilung. Diese Porengröße bewirkt, dass die Abgabegeschwindigkeit des Duftes in dem gewünschten Bereich liegt. Beispielsweise können 50 g einer wenigstens Duftstoff enthaltenen flüssigen Mischung einen Raum einer Größe von 20 m² über einen Zeitraum im Bereich von 1 Woche bis 5 Monate beduften. Zudem wird die Mischung vollständig in den Körper aufgenommen, so dass die Mischung bei Berührung des Körpers nicht austritt. Bei einem Körper mit dieser Porengröße beträgt die Wasseraufnahme mindestens 70 % des Gewichts des Körpers, vorzugsweise mindestens 80 % des Gewichts des Körpers. Dies bedeutet beispielsweise, dass, wenn der Körper ein Gewicht von 300 g hat, die Aufnahmekapazität an Wasser mindestens 210 g, vorzugsweise mindestens 240 g beträgt. Die hohe Aufnahmekapazität des porösen Körpers bewirkt ebenfalls eine lange Beduftungszeit. Bei diesem Beispiel ist zudem die äußere Oberfläche des Körpers vergleichsweise eben, was an dem hohen Anteil an kleinen Poren liegt. Zudem kann während der Formgebung des Grünkörpers ein Relief in die Oberfläche des Körpers eingepresst werden, so dass eine unverwechselbare Form erzeugt wird.

In beiden oben genannten Fällen kann die Porenverteilung für jede Gruppe von Poren mit einer Normalverteilung angenähert werden.

In einem Ausführungsbeispiel enthält der Körper eine gesinterte Keramik, vorzugsweise eine Silikatkeramik. Besonders bevorzugt besteht der Körper vollständig aus dieser gesinterten Keramik.

Erfindungsgemäß ist der Körper wiederverwendbar, vorzugsweise bis zu 10.000 Mal. Die eingefüllte Mischung kann derart gestaltet sein, dass er durch Verdunsten vollständig aus dem Körper entweicht. Alternativ, wenn die Mischung nicht vollständig mittels Verdunsten aus dem Körper entweicht, kann der Körper, um den Körper wiederverwenden zu können, mittels einer Heizeinrichtung erwärmt werden, um den evtl. noch in den Poren vorhandenen Reste vollständig zu entfernen. Die Entfernung erfolgt in diesem Fall mittels Verbrennung. Die Heiztemperatur der Heizeinrichtung liegt im Bereich zwischen 200 °C und 1000 °C, vorzugsweise zwischen 650 °C und 900 °C. Der poröse Körper wird demnach aufbereitet, indem er bei der genannten Temperatur ausgeheizt wird, beispielsweise über einen Zeitraum von 1 Stunde bis 3 Stunden. Die erfindungsgemäße Porengrößenverteilung führt dazu, dass die Wärme der Heizeinrichtung ideal in die Poren eindringen kann, so dass die Mischung rückstandsfrei verbrannt werden kann. Nach der thermischen Behandlung sind somit keine Bestandteile der vorherigen "alten" Mischung mehr vorhanden, welche die Qualität und den Geruch der neuen Befüllung beeinträchtigen würden. Nach der thermischen Behandlung kann der poröse Körper mit einer gewünschten, erfindungsgemäßen Mischung wieder befüllt werden.

Die Heizeinrichtung kann analog zu einem konventionellen Sinterofen aufgebaut sein, der eine (wieder-)verschließbare Heizkammer aufweist. Er kann einen Abzug für ggf. entstehende Gas, wobei eine Abzugsklappe auch zeitweise geschlossen sein kann, und/oder eine Luftzuführungseinrichtung aufweisen.

Der poröse Körper kann jede beliebige dreidimensionale Außen-Form annehmen.

Dies bedeutet, dass die äußeren Flächen des Körpers (bei Vernachlässigung der Unebenheit der Oberfläche wegen der Porigkeit) die genannte Außen-Form bilden. Diese kann mittels Pressen oder Gießen des Grünkörpers vor dem Sintern bzw. durch entsprechende Nachbearbeitung (wie Schneiden und Schleifen) nach den Sintern erzeugt werden. Der Körper kann beispielsweise eine Quader-, Würfel-, Pyramiden-, Zylinder-, Kugel- oder Plattenform aufweisen. Beliebige unregelmäßige Formen, aus obigen Formen abgeleitete Formen sowie Kombinationen aus den oben genannten Formen sind ebenfalls realisierbar. Der Körper kann so dem Einsatzort, beispielsweise einem Gehäuse eines Spenders, angepasst werden. Der poröse Körper kann, da er selbsttragend ist, entweder ohne ein Gehäuse oder mit einer Halterung bzw. einem Gehäuse verwendet werden. Bei einem Einsatz ohne Halterung bzw. Gehäuse kann der poröse Körper gegebenenfalls auf einer Unterlage (z.B. aus Glas, Stein, Keramik, chemisch beständigem Kunststoff (beispielsweise Polypropylen) oder Edelstahl) und an einer beliebigen Stelle in einem Raum platziert werden, um das Beduften dieses Raums zu bewirken.

Wie oben bereits erläutert wurde, ist ein wesentliches Element des Systems zum Beduften von Räumen der poröse Körper. Der Körper ist nach dem Sintern selbsttragend und weist ein poröses, keramisches und wiederverwendbares Material aufweist, wobei das Material des Körpers eine offene Porosität besitzt, die größer als 50% ist und entweder eine bimodale Porengrößenverteilung aufweist, wobei ein erstes Maximum in der Porengrößenverteilung für die erste Gruppe von Poren im Bereich zwischen 1 mm und 3 mm und ein zweites Maximum in der Porengrößenverteilung für die zweite Gruppe von Poren im Bereich zwischen 0,01 mm und 0,3 mm liegt.

Um die erfindungsgemäße Porengrößenverteilung und somit eine hohe Aufnahmekapazität des Körpers zu erzielen, kann dieser nach dem nachfolgend erläuterten Verfahren hergestellt werden. Das Verfahren weist insbesondere die folgenden Schritte auf:
- Erstellung eines Gemischs aus ungebranntem Tonmineral, Wasser sowie Holz-Feinstaub und gegebenenfalls anteilig Holzschnitzeln und
- Sintern des Gemischs bei einer Temperatur zwischen 1000 °C und 1300 °C über einen Zeitraum im Bereich 3 bis 10 Stunden,
wobei das Gemisch vor dem Sintern geformt wird, beispielsweise mittels Pressen, und/oder der Körper nach dem Sintern in die gewünschte Form gebracht wird, beispielsweise mittels Schneiden und/oder Schleifen.

Hierbei wird unter Tonmineral ein Schichtsilikat und ein Silikat mit einer Korngröße < 2 µm verstanden und umfasst mindestens ein Silikat der Gruppe umfassend Zweischichtsilikate, Dreischichtsilikate, Schichtsilikate mit Wechsellagerungsstrukturen, Tonminerale mit Faserstruktur und nichtkristalline Silikate. Zu den Zweischichtsilikaten zählen Kaolin- und Serpentinminerale wie Kaolinit, Dickit, Halloysit sowie Antigorit, Lizardit und Chrysotil. Zu den Dreischichtsilikaten gehören Pyrophyllit und Talk, Minerale der Glimmer-Gruppe wie Muskovit, der Smectit-Gruppe wie Montmorillonit, Beidellit und Saponit, der Hydroglimmer-Gruppe wie Illit und Glaukonit sowie weiterhin Vermiculite und Chlorite. Zu den Wechsellagerungsstrukturen zählen die weitverbreiteten unregelmäßigen Muskovit-Montmorillonit-Mixed-Layer (auch Illit-Smectit-Mixed-Layer). Die Tonminerale mit Faserstruktur sind Sepiolith und Palygorskit. Zu den nichtkristallinen Silikaten zählen des weiteren Allophan und Imogolit. Als Tonmineral werden Quarzsand-Gemische verstanden, deren Zusammensetzung überwiegend SiO₂ und einen Massenanteil von bis zu 20 % Al₂O₃ und bis zu 10 % CaO aufweist. Es können ferner geringe Mengen eines oder mehrere Verbindungen der Gruppe enthaltend TiO₂, Fe₂O₃, MgO, K₂O und Na₂O vorhanden sein. Ein Tonmineral ist ein preiswerter mineralischer Rohstoff und steht in großen Mengen für viele industrielle Zweige zur Verfügung.

Die Holzschnitzel sind vorzugsweise Weichholz-Holzschnitzel, also Holzschnitzel aus Tannen- und/oder Kiefernholz. Sie werden auch als Weichholz-Schweinemehl bezeichnet, da sie auch in der Tierhaltung als Einstreu verwendet werden. Die Holzschnitzel werden auch als unbehandeltes Weichholzsägemehl bezeichnet und sind entrindet, technisch getrocknet und wärmebehandelt. Die für diese Anwendung verwendeten Holzschnitzel aus Weichholz haben eine mittlere Schnitzelgröße, die im Bereich zwischen 1 mm und 5 mm, vorzugsweise zwischen 1 mm und 4 mm liegt.

Der Holz-Feinstaub ist beispielsweise Buchen-Feinstaub, der auch als Buchenlachs bezeichnet wird (z.B. Buchenlachs der Fa. Thomsen Räucherspäne Räucherholz GmbH & Co. KG). Der Holz-Feinstaub hat eine Korngröße von bis zu 0,02 mm.

Um das Gemisch zu erzeugen, werden die Bestandteile in einem Gewichtsverhältnis von ungebranntem Tonmineral zu (Holz-Feinstaub und gegebenenfalls anteilig Holzschnitzel) zu Wasser in dem Gemisch vor dem Sintern 1 : (0,45 bis 0,8) : (0,9 bis 1,2) zugegeben und homogenisiert. Vorzugsweise beträgt das Gewichtsverhältnis von ungebranntem Tonmineral zu (Holz-Feinstaub und gegebenenfalls anteilig Holzschnitzel) zu Wasser in dem Gemisch 1 : (0,5 bis 0,75) : (1 bis 1,2). Das so entstehende Gemisch ist ein heterogenes Gemisch, das durch die Zugabe von Wasser als Suspension oder Gemenge bezeichnet werden kann. Hierbei werden die oben angegebenen Zusammensetzungen des Gemischs so verstanden, dass der Anteil an Holz-Feinstaub und Holzschnitzel zusammengerechnet wird. Wird lediglich Holz-Feinstaub verwendet, so besteht das Gewichtsverhältnisanteil von 0,45 bis 0,8 (vorzugsweise 0,5 bis 0,75) vollständig aus Holz-Feinstaub. Falls auch Holzschnitzel verwendet werden, besteht ein Teil hiervon aus den oben beschriebenen Holzschnitzeln, von dem Holz-Feinstaub ist entsprechend weniger in dem Gemisch vorhanden. Um die oben beschriebene bimodale Porengrößeverteilung zu erreichen, beträgt das Gewichtsverhältnis von Holzschnitzel zu Holz-Feinstaub in dem Gemisch vorzugsweise 1 : (1,2 bis 2). Zum Mischen, beispielsweise über einen Zeitraum von 5 Minuten bis 1 Stunde, vorzugsweise von 10 Minuten bis 45 Minuten, können beispielsweise ein Hub-Kneter oder ein Z-Arm-Mischer verwendet werden.

Anschließend wird das Gemisch bei einer Temperatur zwischen 1000 °C und 1300 °C, vorzugsweise zwischen 1100 °C und 1200 °C über einen Zeitraum im Bereich 3 bis 10 Stunden, vorzugsweise 4 bis 7 Stunden, an Luft unter Normaldruck gesintert.

Vor dem Sintern wird das Gemisch geformt, beispielsweise mittels Pressen oder Gießen. Es wird beispielsweise ein Rammpressverfahren, ein Topfpressverfahren oder ein Waagerecht-Topfpressverfahren verwendet. Hierdurch werden Grünkörper erzeugt, die sich jedoch in Größe und/oder Form durch das Sintern nochmals verändern. In einem Ausführungsbeispiel kann während des Pressens mittels eines Stempels eine Reliefstruktur in die Oberfläche des Körpers, beispielsweise eine obere Oberfläche, eingebracht werden. Diese Reliefstruktur kann eine Einkerbung (Riefe, Rille) und/oder Erhebung in der Form von einem Bild, Zeichen in Form von Buchstaben und Zahlen oder dergl. aufweisen. Die Einkerbung kann vorzugsweise in eine Tiefe von 1,5 cm von der Oberfläche des Körpers reichen. Die Reliefstruktur dient beispielsweise zur Kennzeichnung und zur Verzierung des Körpers, wobei die Reliefstruktur besonders einfach in einen Grünkörper einbringbar ist, der lediglich Holz-Feinstaub in dem oben angegebenen Verhältnis oder höchstens eine kleine Menge an Holzschnitzeln enthält.

Nach dem Sintern kann der Körper beispielsweise mittels Schneiden und/oder Schleifen in die gewünschte Form und/oder Größe gebracht werden. Die Form und/oder die Größe des Körpers wird dem jeweiligen Einsatzzweck und der Dauer des Beduftens angepasst.

Die Mischung mit dem wenigstens einem Duftstoff, welche Bestandteil des Systems ist, setzt sich aus einer Vielzahl von Bestandteilen zusammen, die ein unterschiedliches Verhalten in Bezug auf das Entweichen aus dem porösen Körper aufweisen.

Duftstoff sind dabei jede Form von organischen natürlichen, naturidentischen und synthetischen Duftstoffen. Der Begriff umfasst ätherischen Ölen mit all ihren Bestandteilen, vor allem Terpene und aus dieser Gruppe insbesondere Alkohol-, Glycosid-, Ether-, Aldehyd-, Keton-, Carbonsäure- und Ester-Terpene. Umfasst sind ferner vor allem weitere Angehörige der Klasse der Aromaten, der Ester, der Alkylpyrazine, der Aldehyde und der Ketone.

In einem bevorzugten Ausführungsbeispiel ist die Mischung eine Zusammensetzung bestehend im Wesentlichen aus Duftstoffen in Form von Top-Noten und Herz-Noten. Vorzugsweise besteht mindestens ein Anteil von 80 Gew.-%, vorzugsweise ein Anteil von mindestens 90 Gew.%, der Mischung aus Top-Noten und Herz-Noten. Hierbei sind Top-Noten Bestandteile der Mischung, die sich sehr schnell verflüchtigen. Herz-Noten verflüchtigen sich langsamer und bilden die "Seele" des von der Mischung erzeugten Duftes. Sogenannte Basis-Noten, die sehr lange Zeit benötigen, um sich zu verflüchtigen, sind nur zu einem kleinen Anteil (weniger als 20 Gew.-%, vorzugsweise weniger als 10 Gew.%, der Mischung) in der Zusammensetzung enthalten.

Beispiele für ätherische Öle, die für Top-Noten verwendet werden können, sind ätherische Öle aus Bergamotte, Blutorange, Cananga, Christrose, Clemetine, Erdbeere, Pfirsich, rote Beeren, Eukalyptus, Gingergras, Grapefruit, Immortelle, Kamille, Krause Minze, Limette, Majoran, Melisse, Minze, Myrte, Palmarosa, Rosenholz, Rosmarin, Spearmint, Wacholder, Wintergrün, Zedernblätter, Zimt, Zypresse.

Beispiele ätherischer Öle für Herz-Noten sind ätherische Öle aus Anis, Bayöl, Bitter Orange, Cajeput, Eisenkraut, Fenchel, Guajakholz, Honig, Immortelle, Iris, Kalmus, Karotte, Latschenkiefer, Lavendel, Lemongras, Macisblüte, Mandarine, Mimose, Moschus, Muskatnuss, Niaouli, Petitgrain, Pfefferminze, Pfeffer, Salbei, süße Orange, Teebaum, Vetiver, Ysop, Zedernholz, Zypresse.

Ätherische Öle, die als Basis-Noten verwendet werden können, sind ätherische Öle aus Asant, Balsamterpentin, Bayöl, Birkenrinde, Damaszener Rose, Elemi, Galbanum, Geranium, Ho-Blätter, Ingwer, Jasmin, Mastix Pistazie, Muskatellersalbei, Moschus, Myrrhe, Neroli, Nelke, Patchouli, Sandelholz, Teebaum, Tonkabohnen, Vanille, Weihrauch, Zedernholz, Zibetkatze, Ylang Ylang.

Es können jeweils mindestens eine der obigen Noten oder mehrere der obigen Noten verwendet werden.

Erfindungsgemäß weist die Mischung zudem einen niedrigen Flammpunkt auf, der kleiner oder gleich 120 °C, vorzugsweise kleiner oder gleich 110 °C, besonders bevorzugt kleiner oder gleich 100 °C ist. Der Flammpunkt wird bestimmt mittels der Methode nach Pensky-Martens (DIN 51758, EN 22719) bei Normaldruck (1013 hPa). Mischungen des erfindungsgemäßen Systems mit dem genannten niedrigen Flammpunkt zeichnen sich dadurch aus, dass sie über einen bestimmten, vergleichsweise kurzen Zeitraum rückstandsfrei verdampfen, da sie einen geringen Dampfdruck aufweisen, so dass der poröse Körper wiederverwendet werden kann und der Geruch einer neuen Befüllung des porösen Körpers nicht durch Restprodukte der vorherigen Befüllung beeinträchtigt wird. Zudem kann das gesamte poröse Volumen des Körpers bei der Wiederbefüllung mittels einer anderen Mischung genutzt werden.

In einem Ausführungsbeispiel ist der Flammpunkt der Mischung größer als 60 °C. Bei einem Flammpunkt kleiner oder gleich 60 °C werden Mischungen nach EU-Verordnung 1272/2008/EG als entzündbare Flüssigkeiten der Kategorie 1 (extrem entzündbar), 2 (leicht entzündbar) und 3 (entzündbar) eingestuft und es müssen beim Transport, Lagerung und Verwendung entsprechende Maßnahmen getroffen werden. Dieser zusätzliche Aufwand soll bei der Mischung für das erfindungsgemäße System vermieden werden.

Die Mischung des erfindungsgemäßen Systems setzt sich zusammen aus einer Vielzahl von einzelnen Verbindungen, die in unterschiedlichen Konzentrationen in der Mischung enthalten sind.

Das erfindungsgemäße System ist besonders umweltfreundlich, da der poröse Körper wiederverwendet und wieder neu befüllt werden kann. Die geringe Umweltbelastung des erfindungsgemäßen Systems zum Beduften eines Raumes rührt auch daher, dass die Aufnahmekapazität des porösen Körpers, wie oben beschrieben, vergleichsweise groß ist, so dass der poröse Körper seine Duftwirkung über den oben angegebenen, vergleichsweise langen Zeitraum in dem jeweiligen Raum entfalten kann.

In dem erfindungsgemäßen System kann zusätzlich ein Mischungs-Spender (im Folgenden kurz Spender) vorgesehen sein, der ein Gehäuse zur Anordnung des oben beschriebenen porösen Körpers des Systems aufweist. Die Anordnung des Körpers erfolgt dabei derart, dass Duftstoffe in die Luft des umgebenden Raumes entweichen können.

In einem Ausführungsbeispiel ist das Gehäuse derart gestaltet, dass es ein Auswechseln des Körpers und/oder ein Wiederbefüllen des Körpers mit einer anderen Mischung ermöglicht.

In einem Ausführungsbeispiel ist das Gehäuse dosenartig gestaltet und die Anordnung des Körpers in dem Gehäuse erfolgt derart, dass die Mischung zumindest in einem geöffneten Zustand verdampfen und den umgebende Raum entweichen kann. Dies bedeutet, dass die gasförmige Mischung sich mit der Luft des Raumes mischt. Beispielsweise kann das Gehäuse als Hohlkörper (z.B. Hohlzylinder, Hohlkegel, Hohlquader, Hohlwürfel) ausgebildet sein, wobei der keramische Körper in dem Hohlraum des Gehäuses aufgenommen ist. Das Gehäuse kann beispielsweise aus Edelstahl, Glas, Stein oder Keramik oder einer Kombination einer oder mehrerer dieser Materialien bestehen. Für das Entweichen der Mischung aus dem Spender kann das Gehäuse beispielsweise permanent durchgehende Öffnungen oder einen abnehmbaren, drehbaren und/oder verschieblichen Deckel aufweisen. In einem Ausführungsbeispiel kann der Deckel des Gehäuses eine erste Position einnehmen, in der der Deckel das Gehäuse verschließt, und eine zweite Position, in der der Deckel gegenüber der ersten Position gedreht und/oder verschoben ist, sodass das Gehäuse in der zweiten Position zumindest teilweise unverschlossen ist. In der ersten Position ist somit das Gehäuse verschlossen und die gasförmige, verdampfte Mischung kann nicht nach außen entweichen. In der zweiten Position bildet sich durch das Verschieben und/oder Verdrehen des Deckels eine Öffnung, durch welche die Mischung entweichen kann. In einem Ausführungsbeispiel wird der Deckel vollständig entfernt.

Im Fall der Ausgestaltung des Gehäuses mit permanenten durchgehenden Öffnungen (z.B. kreisrunden, elliptischen und/oder eckigen Öffnungen) in einer oberen Wand und/oder einer Seitenwand des Gehäuses, bei der kein drehbarer und/der verschieblicher Deckel vorgesehen ist, kann das Gehäuse vor der Anwendung mittels einer entsprechend luftdichten Verpackung (z.B. einer Kunststoffverpackung) verschlossen werden.

Oben wurde bereits beschrieben, dass erfindungsgemäß außerdem ein Verfahren zur Aufbereitung des Systems durchgeführt werden kann, bei dem der poröse Körper nach der Verwendung in einer Heizeinrichtung bei einer Temperatur zwischen 200 °C und 1000 °C, vorzugsweise zwischen 200 °C und 650 °C, ausgeheizt wird, um ggf. die Mischung vollständig aus dem Körper zu entfernen. Anschließend kann der poröse Körper wieder mit einer Mischung befüllt werden, so dass dieser in dem Körper aufgenommen wird. Hierdurch wird eine Wiederverwendbarkeit des Systems realisiert, wobei nacheinander gleiche oder unterschiedliche Mischungen verwendet werden können. Durch die vollständige Entfernung der Mischung kann die neue Mischung einen (ihren) Geruch entfalten, ohne dass dieser von dem vorher verwendeten Duftstoffen beeinträchtigt wird.

Weitere Merkmale, Vorteile und Anwendungsmöglichkeiten der Erfindung ergeben sich auch aus der nachfolgenden Beschreibung von Ausführungsbeispielen eines erfindungsgemäßen Systems und den Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den Ansprüchen oder deren Rückbezüge.

Es zeigen schematisch:
- Fig. 1 und 2: ein erstes Ausführungsbeispiel eines erfindungsgemäßen Systems in einer perspektivischen Ansicht von der Seite mit Deckel (Fig. 1) und ohne Deckel (Fig. 2),
- Fig. 3 und 4: ein zweites Ausführungsbeispiel eines erfindungsgemäßen Systems in einer perspektivischen Ansicht von der Seite mit Deckel (Fig. 4) und ohne Deckel (Fig. 3),
- Fig. 5 bis 8: ein drittes Ausführungsbeispiel eines erfindungsgemäßen Systems, wobei Fig. 5 den Körper in einer perspektivischen Ansicht von der Seite, Fig. 6 das Gehäuse in einer perspektivischen Ansicht von der Seite, Fig. 7 das Gehäuse in einer Ansicht von oben und Fig. 8 das Gehäuse in einer perspektivischen Ansicht von unten zeigt,
- Fig. 9 und 10: das dritte Ausführungsbeispiel in unterschiedlichen Varianten des Gehäusedesigns in einer perspektivischen Ansicht von der Seite und
- Fig. 11: den Körper eines weiteren Ausführungsbeispiels des erfindungsgemäßen Systems in einer Teil-Ansicht von oben.

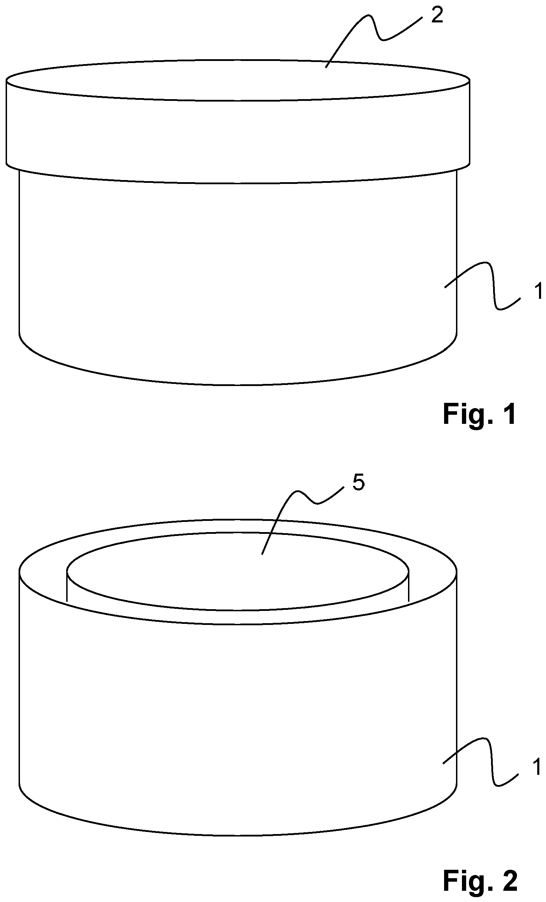

Ein erstes Ausführungsbeispiel eines erfindungsgemäßen Systems ist in Fig. 1 und 2 dargestellt. Das System weist ein hohlzylinderförmiges, unten verschlossenes, dosenförmiges Gehäuse 1 mit einem oben angeordneten, kreisförmigen und abnehmbaren Deckel 2 auf, der das Innere des Gehäuses 1 von der Umgebung abschließt. In der Zeichnung der Fig. 2 ist - bei entferntem Deckel 2 - das Innere des Gehäuses 1 zu erkennen. Dort ist ein zylinderförmiger, selbsttragender, fester und poröser Körper 5 angeordnet. Im Inneren des Körpers befindet sich die flüssige Mischung mit wenigstens einem Duftstoff, der bei geöffnetem Deckel 2 durch Verdunsten in den umgebenden Raum abgegeben wird und diesen hierdurch beduftet. Bei geschlossenem Deckel 2 kann die Mischung nicht entweichen, da der Deckel 2 das Gehäuse 1 mit seinem umkragenden Rand luftdicht verschließt. Bei geöffnetem Deckel 2 kann zudem der Körper 5 entnommen werden, um mittels Ausheizen in einer Heizeinrichtung von Mischungsrückständen befreit und/oder mit einer neuen, anders duftenden Mischung aufgefüllt zu werden.

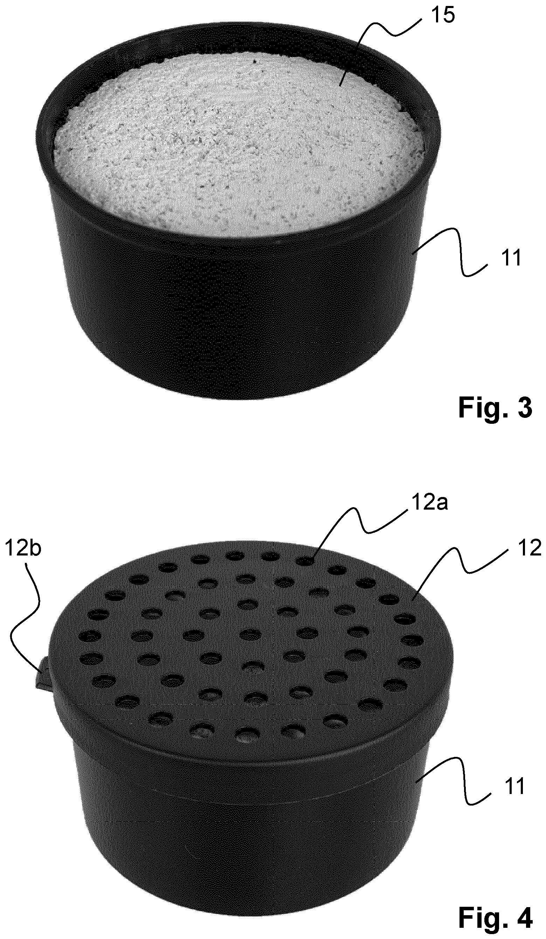

Bei dem in den Fig. 3 und 4 dargestellten zweiten Ausführungsbeispiel ist analog zum ersten Ausführungsbeispiel ein hohlzylinderförmiges, unten verschlossenes Gehäuse 11 vorgesehen, das mit einem oben angeordneten, abnehmbaren Deckel 12 verschlossen ist. Im Innern des Gehäuses 11 ist der zylinderförmige, feste, selbsttragende und poröse Körper 15 mit der in dem Körper aufgenommenen Mischung angeordnet. Der Deckel 12 weist eine Vielzahl von kreisrunden, durchgehenden Öffnungen 12a auf, die es auch im verschlossenen Zustand erlauben, dass die Mischung in den umliegenden Raum zum Beduften entweicht. Der Deckel 12 weist außerdem an dem unteren Rand des umkragenden Randes eine in radiale Richtung (bezogen auf die Achse, die sich durch das Gehäuse 11 bzw. den Körper 15 erstreckt) abstehende Lasche 12b auf, welche ein einfaches Ergreifen und Abziehen des Deckels 12 erlaubt.

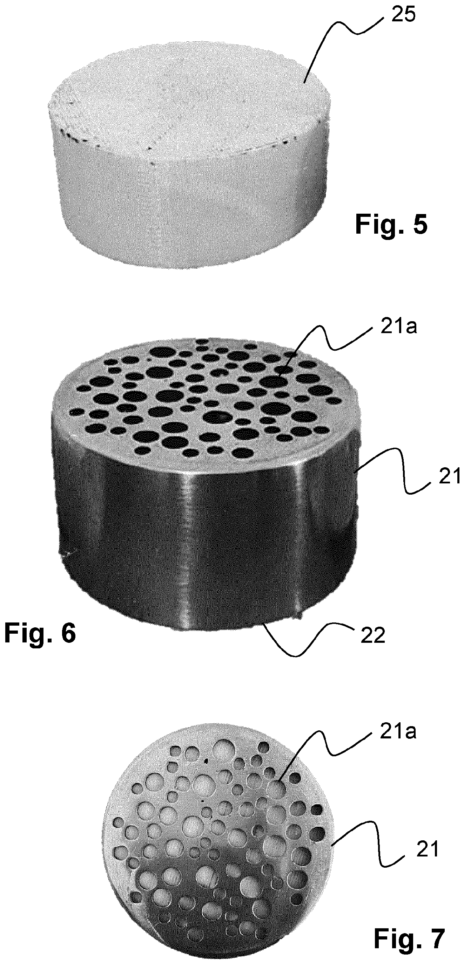

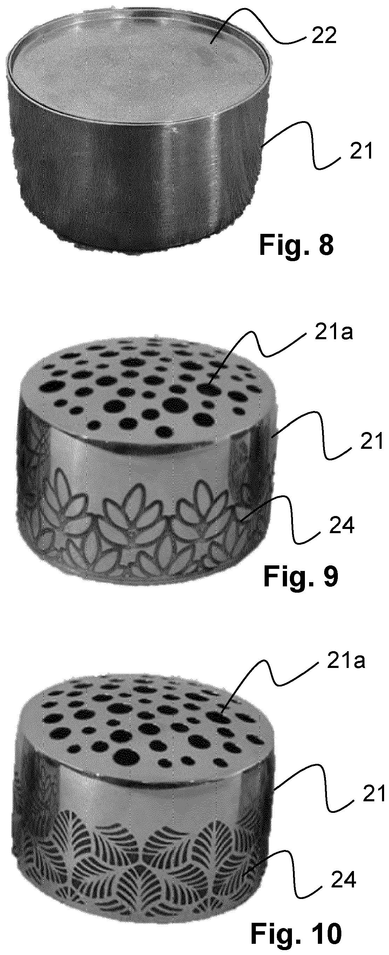

Das dritte, in den Fig. 5 bis 8 dargestellte Ausführungsbeispiel besitzt ebenfalls ein hohlzylinderförmiges Gehäuse 21, das oben verschlossen ist, in der Oberseite jedoch eine Vielzahl von runden oder ovalen durchgehenden Öffnungen 21a aufweist, die einen unterschiedlichen Durchmesser besitzen (siehe Ansicht von oben der Fig. 7) und durch die aus dem Körper 25 verdunstende Mischung in den umgebenden Raum entweichen kann. Bei diesem Ausführungsbeispiel ist an der Unterseite ein Deckel 22 angeordnet, der das Gehäuse 21 fest und auf dieser Seite auch luftdicht verschließt, jedoch zur Entnahme des Körpers 25 entfernt werden kann. Der selbsttragende, keramische, feste und poröse Körper 25 ist in Fig. 5 ohne das Gehäuse 21 dargestellt. Der Körper 25 befindet sich, wie Fig. 7 zeigt, im Inneren des Gehäuses 21, da die Oberseite des Körpers durch die Öffnungen 21a sichtbar ist. Die Figuren 9 und 10 zeigen zwei Varianten des dritten Ausführungsbeispiels mit verschiedenen, an der Mantelfläche des Gehäuses 21 angeordneten Verzierungen. Diese können als Gravuren, farbigen Aufdruck oder dergl. gestaltet sein.

Die Mantelfläche kann zusätzlich oder alternativ bei den obigen Ausführungsbeispielen mit durchgehenden Öffnungen zum Entweichen der Mischung versehen sein.

Das Gehäuse 1, 11, 21 der oben erläuterten Ausführungsbeispiele eines erfindungsgemäßen Systems besteht beispielsweise aus Edelstahl oder Polypropylen.

Der zylindrische poröse Körper 5, 15, 25 zur Aufnahme der Mischung kann beispielsweise nach folgendem Verfahren hergestellt werden. Zuerst wird ein Gemisch aus 1 kg Tonmineral (z.B. mit der Zusammensetzung und dem Massenanteil SiO₂ 73,9 %, TiO₂ 0,7 %, Al₂O₃ 17,3 %, Fe₂O₃ 0,6 %, CaO 6,0 %, MgO 0,2 %, K₂O 1,2 % und Na₂O 0,2 %), 250 g Weichholzspäne (Schweinemehl) und 300 g bis 500 g Buchenlachs, beides z.B. von der Fa. Thomsen Räucherspäne Räucherholz GmbH & Co. KG und 1 bis 1,1 kg Wasser mittels eines Z-Arm-Mischers hergestellt. Die Bestandteile werden ca. 20 Minuten vermischt. Anschließend werden mithilfe einer Topfpresse aus dem Gemisch zylinderförmige Grünkörper erzeugt. Diese Grünkörper werden anschließend bei einer Temperatur von 1150 °C bis 1200 °C über 4 bis 7 Stunden an Luft unter Normaldruck gesintert. Falls erforderlich können die gesinterten Körper anschließend mittels Schleifen oder Schneiden nachgeformt werden, bis der jeweilige gesinterte Körper die benötigten Abmessungen und/oder Form aufweist.

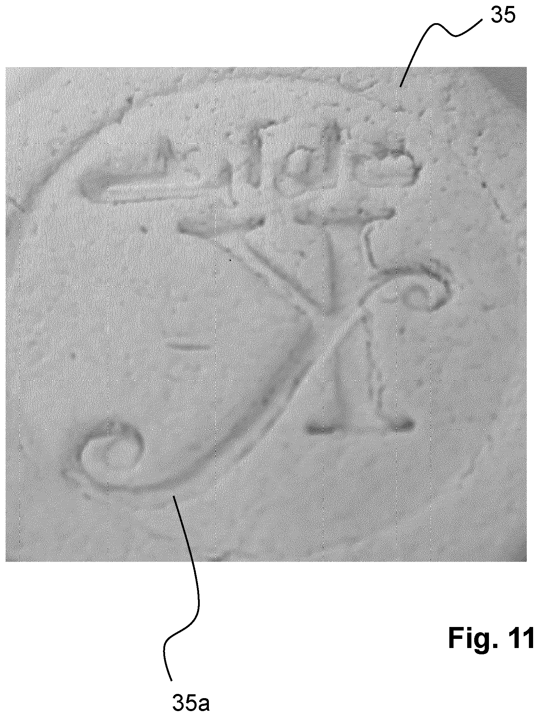

Bei einem Ausführungsbeispiel des Körpers, das in Fig. 11 dargestellt ist, kann während der Herstellung des Grünkörpers in einer Presse (z.B. einer Topfpresse) mittels eines entsprechend geformten Stempels eine Reliefstruktur in der Oberfläche des Körpers eingepresst werden. Fig. 11 zeigt ein Beispiel für einen Körper 35, für dessen Herstellung nur Buchenlachs verwendet wurde (keine Weichholzspäne), mit einer Reliefstruktur 35a in Form einer Vertiefung bestehend aus verschiedenen Schriftzeichen in der Oberfläche des Körpers 35. Die Vertiefung reicht wenige Millimeter in die Oberfläche des Körpers 35. Die Reliefstruktur bleibt auch während des Sinterns erhalten, kann sich hinsichtlich ihrer Abmessungen analog zum Körper während des Sinterns verändern.

Nachdem der fertige Körper in einem Spender mit einem Gehäuse 21 nach Fig. 5 bis 8 platziert wurde, wird noch eine bestimmte, vorgegebene Menge an flüssiger Mischung (z. B. 50 g) mit wenigstens einem Duftstoff auf den Körper gegeben, so dass die Mischung in den Körper aufgenommen wird. Anschließend kann das Gehäuse 21 unten mit dem Deckel 22 verschlossen werden. Das fertiggestellte System wird anschließend luftdicht verpackt, so dass die Mischung mit wenigstens einem Duftstoff nicht vor der bestimmungsgemäßen Verwendung entweichen kann.

Ein Beispiel für eine Mischung, welche eine Reihe von Duftstoffen enthält, ist eine Mischung "Fresh Cotton". Diese Mischung weist einen Flammpunkt von 91 °C auf (bei Normaldruck) und ist daher sehr gut für die Anwendung zum Beduften eines Raumes und zur Verwendung mit dem oben beschriebenen porösen Körper geeignet. Die Mischung enthält einen verhältnismäßig hohen Anteil an Acetaten, welche cyclisch, aromatisch und aliphatisch mit mindestens 6 C-Atomen ausgebildet sind

| **CAS-Nr.** | **Name des Bestandteils** | **Anteil (Gew.-%)** |
|---|---|---|
| **140-11-4** | BENZYL ACETATE | 10 - 25 |
| **32210-23-4** | 4-tert-Butylcyclohexyl acetate | 10 - 25 |
| **58430-94-7** | 3,5,5-Trimethylhexyl acetate | 10 - 25 |
| **115-95-7** | Linalyl acetate | 5 - 10 |
| **142-92-7** | N-HEXYL ACETATE | 1 - 10 |
| **18479-58-8** | 2,6-DIMETHYL-7-OCTEN-2-OL | 1 - 5 |
| **110-98-5** | 1,1'-OXYDIPROPANOL | 1 - 5 |
| **112-31-2** | 1-DECANAL | 1 - 5 |
| **88-41-5** | 2-TERTIARY-BUTYLCYCLOHEXYL ACETATE | 1 - 5 |
| **79-77-6** | (E)-4-(2,6,6-trimethylcyclohex-1-en-1-yl)-but-3-en-2-one | 1 - 5 |
| **17511-60-3** | TRICYCLO DECENYL PROPIONATE | 1 - 5 |
| **2500-83-6** | TRICYCLO DECENYL ACETATE | 1 - 5 |
| **101-84-8** | DIPHENYL ETHER | 1 - 5 |
| **80-26-2** | P-MENTH-1-EN-8-YL aCETATE | 1 - 5 |
| **81786-73-4** | Acetyl diisoamylene, Z | 1 - 5 |
| **51566-62-2** | 3,7-dimethyloct-6-enenitrile | 0,1 - 5 |
| **706-14-9** | gamma-Decalactone | 0,1 - 5 |
| **110-41-8** | 2-Methylundecanal | 0,1 - 1 |
| **14765-30-1** | 2-(1-METHYLPROPYL)CYCLOHEXANONE | 0,1 - 1 |
| **470-82-6** | 1,8-Cineole | 0,1 - 1 |
| **67634-15-5** | A-DIMETHYL HYDROCINNAMALDEHYDE | 0,1 - 1 |
| **93-92-5** | METHYL PHENYL CARBINYL ACETATE | 0,1 - 1 |
| **1191-16-8** | 3-Methyl-2-butenyl acetate | 0,1 - 1 |
| **124-13-0** | 1-OCTANAL | 0,1 - 1 |
| **67634-00-8** | ISO-AMYL OXYACETIC ACID ALLYLESTER | 0,1 - 1 |
| **85-91-6** | Methyl N-methylanthranilate | 0,1 - 1 |
| **137-03-1** | 2-N-HEPTYL CYCLOPENTANONE | 0,1 - 1 |
| **106-72-9** | 2,6-DIMETHYL-5-HEPTENAL | 0,1 - 1 |
| **93-04-9** | 2-Methoxynaphthalene | 0,1 - 1 |
| **141-13-9** | 2,6,10-Trimethyl-9-undecenal | 0,1 - 1 |
| **928-96-1** | BETA GAMMA HEXENOL EXTRA | 0,1 - 1 |
| **54464-57-2** | 7-ACETYL-(1,8)-OCTAHYDRO-1,1,6,7-TETRAM E-THYLNAPTHALENE | 0,1 - 1 |
| **18127-01-0** | 3-(4-TERTBUTYLPHENYL)PROPANAL | 0,1 - 1 |
| **19870-74-7** | CERDYL METHYL ETHER | 0,1 - 1 |
| **68039-49-6** | 2,4-DIMETHYLCYCLOHEXENE-3-CARBALDE-HYDE | 0,1 - 1 |
| **3681-71-8** | HEXENYL ACETATE CIS-3 | 0,1 - 1 |
| **70788-30-6** | 1-(2,2,6-trimethylcyclohexyl)hexan-3-ol | 0,1 - 1 |
| **106185-75-5** | 2-ETHYL-4-(2,2,3-TRIMETHYL-3-CYCLOPENTEN-1-YL)-2-BUTEN-1-OL | 0,1 - 1 |
| **151-05-3** | APLHA, ALPHA-DIMETHYL PHENETHYL ACETATE | 0,1 - 1 |
| **67634-20-2** | Tricyclodecenyl-8-isobutyrate | 0,1 - 1 |
| **13019-22-2** | 9-DECENOL | 0,1 - 1 |
| **99-49-0** | CARVONE | 0,1 - 1 |
| **68901-15-5** | ACETIC ACID, (CYCLOHEXYLOXY):ALLYL ESTER | 0,1 - 1 |
| **5986-55-0** | Patchouli alcohol (NATURAL CONSTITUENT) | 0,1 - 1 |
| **93-08-3** | Methyl beta-naphthyl ketone | 0,1 - 1 |
| **4707-47-5** | Methyl atrarate | 0,1 - 1 |
| **87-44-5** | CARYOPHELENE CRUDE | 0,1 - 1 |
| **79-78-7** | ALLYL IONONE | 0,1 - 1 |
| **87731-18-8** | CYCLOOCTEN-4 AND 5-YL METHYL CARBONATE | 0,1 - 1 |
| **110-27-0** | Isopropyl myristate | 0,1 - 1 |
| **122-03-2** | CUMIN ALDEHYDE | 0,1 - 1 |
| **65442-31-1** | 2-(2-METHYLPROPYL)-QUINOLINE | 0,1 - 1 |
| **2277-19-2** | CIS 6 NONENAL | 0,1 - 1 |
| **35854-86-5** | CIS 6 NONENOL | 0,1 - 1 |

Hierbei wird die obige Zusammensetzung der Mischung derart gestaltet, dass alle Bestandteile zusammen 100 Gew.% ergeben.

Eine Menge von 50 g der oben angegebenen Mischung "Fresh Cotton" ist in einem 20 m² großen Raum nach zwei bis drei Monaten vollständig aus dem porösen Körper entwichen. Es sind keine Rückstände vorhanden.

Eine weitere Mischung, die in dem oben beschriebenen Keramik-Körper verwendet werden kann, wird als "Morning Dew" bezeichnet. Diese Mischung hat die folgende Zusammensetzung und besitzt einen Flammpunkt, der zwischen 100 °C und 120 °C liegt.

| **CAS-Nr.** | **Name des Bestandteils** | **Anteil (Gew.-%)** |
|---|---|---|
| **112-54-9** | DODECANAL | 0,1 - 1 |
| **67634-00-8** | ISO-AMYL OXYACETIC ACID ALLYLESTER | 1 - 5 |
| **2705-87-5** | ALLYL CYCLOHEXANEPROPIONATE | 1 - 5 |
| **7493-74-5** | ACETIC ACID, PHENOXY ALLYL ESTER | 1 - 5 |
| 140-11-4 | BENZYL ACETATE | 1 - 5 |
| **122-63-4** | BENZYL N-PROPANOATE | geringer Anteil |
| **99-49-0** | CARVONE | 0,1 - 1 |
| **2500-83-6** | TRICYCLO DECENYL ACETATE | geringer Anteil |
| **103-95-7** | 2-METHYL-4-ISOPROPYLDIHYDROCINNAMALDE-HYDE | 1 - 5 |
| **68901-15-5** | ACETIC ACID, (CYCLOHEXYLOXY):ALLYL ESTER | 0,1 - 1 |
| **18479-58-8** | 2,6-DIMETHYL-7-OCTEN-2-OL | 1 - 5 |
| **10094-34-5** | ALPHA,ALPHA-DIMETHYL PHENETHYL BUTYRATE | 1 - 5 |
| **151-05-3** | APLHA, ALPHA-DIMETHYL PHENETHYL ACETATE | geringer Anteil |
| **110-98-5** | 1,1'-OXYDIPROPANOL | geringer Anteil |
| **105-95-3** | TRICANEDIOIC ACID: CYCLI | geringer Anteil |
| **67634-15-5** | A-DIMETHYL HYDROCINNAMALDEHYDE | 1 - 5 |
| **125109-85-5** | 3-(3-ISOPROPYLPHENYL)BUTANAL | geringer Anteil |
| **63500-71-0** | 2-(2-METHYL PROPYL)-4-HYDROXY-4-METHYL TETRAHYDROPYRAN | 1 - 5 |
| **67634-20-2** | Tricyclodecenyl-8-isobutyrate | 0,1 - 1 |
| **105-87-3** | 3,7-dimethyl-2,6-octadien-1-yl-acetate | 1 - 5 |
| **125-12-2** | EXO-1,7,7TRIMETHYLBICYCLO(2.2.1)HEPT-2YL ACETATE | geringer Anteil |
| **1335-66-6** | 3-CYCLOHEXENE-1-CARBOXALDEHYDE, 2,4,6-TRIMETHYL | 0,1 - 1 |
| **38285-49-3** | 4-ACETOXY-3-PENTYLTETRAHYDROPYRAN | geringer Anteil |
| **706-14-9** | gamma-Decalactone | geringer Anteil |
| **67633-96-9** | cis-3-hexenyl methyl carbonate | 0,1 - 1 |
| **2216-51-5** | DL-MENTHOL | geringer Anteil |
| **141-12-8** | Neryl acetate | geringer Anteil |
| **103-60-6** | 2-Phenoxyethyl isobutyrate | geringer Anteil |
| **103-48-0** | 2-phenylethyl 2-methylpropanoate | geringer Anteil |
| **93-92-5** | METHYL PHENYL CARBINYL ACETATE | geringer Anteil |
| **78-69-3** | 3-OCTANOL, 3,7-DIMETHYL- Octanol | 10 - 25 |
| **81782-77-6** | 4-METHYL-3-DECEN-5-OL | 5 - 10 |
| **65443-14-3** | 2,2,5-Trimethyl-5-pentylcyclopentanone | geringer Anteil |

Hierbei wird die obige Zusammensetzung der Mischung derart gestaltet, dass alle Bestandteile zusammen 100 Gew.% ergeben.

Eine Menge von 50 g der oben angegebenen Duftmischung "Morning Dew" ist in einem 20 m² großen Raum nach zwei bis drei Monaten vollständig aus dem porösen Körper entwichen. Es sind keine Rückstände vorhanden.

Eine der oben dargestellten Mischungen kann in dem obigen System verwendet werden. Zur Anwendung wird die luftdichte Verpackung entfernt und der Spender wird an einem gewünschten Ort in einem Raum aufgestellt. Dort entweicht die Mischung langsam durch die Öffnungen 21a des Gehäuses 21 in die Luft des Raumes und beduftet diesen. Nach einigen Wochen bis 4 Monaten ist entweder die Mischung vollständig aus dem Körper 25 entwichen oder es sind noch Rückstände in dem Körper 25 enthalten. In dem zweiten Fall wird der Körper durch Entfernen des Deckels an der Unterseite des Gehäuses 21 aus dem Gehäuse 21 entnommen und in einem Sinterofen bei einer Temperatur zwischen 650 °C und 900 °C an Luft getempert (ausgeheizt), um die restliche Menge an in dem Körper 25 verbliebenen Mischung zu entfernen. Anschließend kann der Körper 25 neu mit einer (anderen oder gleichen) Mischung befüllt werden. Bei einem vollständigen Verdunsten der Duftmischung aus dem Körper 25 ist ein solches Ausheizen nicht notwendig und der Körper kann sofort wieder befüllt werden. Anschließend wird das Gehäuse 21 durch Schließen des Deckels 22 wieder verschlossen.

Durch die Wiederverwendbarkeit des Systems (ca. 10.000 Mal möglich) können beträchtliche Mengen an Verpackungen und Behältern gespart werden, die sonst nach jedem Aufbrauchen einer vorgegebenen Duftstoffmenge weggeworfen worden wären. Daher trägt das erfindungsgemäße System zum Umweltschutz bei.

## Patentansprüche

1. System zum Beduften von Räumen mit einem festen Körper (5, 15, 25, 35) und einer flüssigen Mischung, wobei der Körper (5, 15, 25, 35) zur Anordnung und zum Verdunsten der flüssigen Mischung dient, welche mindestens einen Duftstoff enthält, wobei der Körper (5, 15, 25, 35) ein poröses, keramisches und wiederverwendbares Material aufweist und das Material des Körpers (5, 15, 25, 35) eine offene Porosität besitzt, die größer als 50% ist, und eine bimodale Porengrößenverteilung aufweist, wobei ein erstes Maximum in der Porengrößenverteilung für die erste Gruppe von Poren im Bereich zwischen 1 mm und 3 mm und ein zweites Maximum in der Porengrößenverteilung für die zweite Gruppe von Poren im Bereich zwischen 0,01 mm und 0,3 mm liegt, und wobei die Mischung einen Flammpunkt aufweist, der gleich oder kleiner als 120 °C ist.

2. System nach Anspruch 1, **dadurch gekennzeichnet, dass** die Mischung eine Zusammensetzung bestehend im Wesentlichen aus Top-Noten und Herz-Noten bildet.

3. System nach einem der vorhergehenden Ansprüche, **dadurch gekennzeichnet, dass** der Körper (5, 15, 25, 35) zur vollständigen Entfernung der Mischung mittels einer Heizeinrichtung erwärmbar ist, wobei die Heiztemperatur im Bereich zwischen 200 °C und 1000 °C liegt.

4. System nach Anspruch 1, **dadurch gekennzeichnet, dass** das keramische Material des Körpers (5, 15, 25, 35) eine Silikat-Keramik aufweist.

5. System nach einem der Ansprüche 1 und 4, **dadurch gekennzeichnet, dass** der Körper (5, 15, 25, 35) auf der Oberfläche eine Reliefstruktur mit einer Vertiefung und/oder Erhebung aufweist, wobei die Einkerbung vorzugsweise eine Tiefe von bis zu 1,5 cm aufweist.

6. System nach einem der Ansprüche 1 bis 3, zusätzlich aufweisend einen Spender mit einem Gehäuse (1, 11, 21) zur Anordnung des Körpers (5, 15, 25, 35), wobei das Gehäuse dosenartig gestaltet ist und die Anordnung des Körpers (5, 15, 25, 35) in dem Gehäuse derart erfolgt, dass die Mischung zumindest in einem geöffneten Zustand des Gehäuses verdampfen und in den umgebenden Raum entweichen kann.

7. Verfahren zur Aufbereitung des Systems nach einem der Ansprüche 1 bis 3, **dadurch gekennzeichnet, dass** der poröse Körper (5, 15, 25, 35) nach der Verwendung in einer Heizeinrichtung bei einer Temperatur zwischen 200 °C und 1000 °C, vorzugsweise zwischen 200 °C und 650 °C, ausgeheizt wird, wobei der Körper (5, 15, 25, 35) nach dem Ausheizen beispielsweise wieder mit einer Mischung derart befüllt wird, dass diese in den Körper (5, 15, 25, 35) aufgenommen wird.

## Claims

1. A system for scenting rooms comprising a solid body (5, 15, 25, 35) and a liquid composition, said body (5, 15, 25, 35) serving to dispose and evaporate said liquid composition containing at least one fragrance, wherein said body (5, 15, 25, 35) comprises a porous, ceramic and reusable material and the material of said body (5, 15, 25, 35) has an open porosity which is greater than 50%, and has a bimodal pore size distribution, wherein a first maximum in the pore size distribution for the first group of pores is in the range between 1 mm and 3 mm and a second maximum in the pore size distribution for the second group of pores is in the range between 0.01 mm and 0.3 mm, and wherein the composition has a flash point which is equal to or less than 120 °C.

2. System according to claim 1, **characterized in that** the composition forms a combination consisting essentially of top notes and heart notes.

3. System according to any one of the preceding claims, **characterized in that** the body (5, 15, 25, 35) is heatable by means of a heating device for complete removal of the composition, wherein the heating temperature is in the range between 200 °C and 1000 °C.

4. System according to claim 1, **characterized in that** the ceramic material of the body (5, 15, 25, 35) comprises a silicate ceramic.

5. System according to any one of claims 1 and 4, **characterized in that** the body (5, 15, 25, 35) has on the surface a relief structure with an indentation and/or elevation, wherein the indentation has preferably a depth of up to 1.5 cm.

6. System according to any one of claims 1 to 3, additionally comprising a dispenser with a housing (1, 11, 21) for arranging the composition (5, 15, 25, 35), wherein the housing is shaped like a box and the arrangement of the composition (5, 15, 25, 35) in the housing is such that the composition can evaporate and escape into the surrounding space at least in an opened state of the housing.

7. Method of preparing the system according to any one of claims 1 to 3, **characterized in that** the porous body (5, 15, 25, 35) is baked out after use in a heating device at a temperature between 200 °C and 1000 °C, preferably between 200 °C and 650 °C, wherein the body (5, 15, 25, 35) after baking out is, for example, refilled with a composition in such a way that the composition is received in the body (5, 15, 25, 35).

## Revendications

1. Système, destiné à parfumer des espaces, pourvu d'un corps (5, 15, 25, 35) solide et d'un mélange liquide, le corps (5, 15, 25, 35) servant à y placer et à faire évaporer le mélange liquide, lequel contient au moins un parfum, le corps (5, 15, 25, 35) comportant une matière céramique poreuse et recyclable et la matière du corps (5, 15, 25, 35) présentant une porosité ouverte, qui est supérieure à 50 % et une distribution bimodale de la taille des pores, un premier maximum dans la distribution de la taille des pores pour le premier groupe de pores se situant dans la fourchette comprise entre 1 mm et 3 mm et un deuxième maximum dans la distribution de la taille des portes pour le deuxième groupe de pores se situant dans la fourchette comprise entre 0,01 mm et 0,3 mm, et le mélange faisant preuve d'un point d'éclair qui est inférieur ou égal à 120 °C.

2. Système selon la revendication 1, **caractérisé en ce que** le mélange consiste dans une composition constituée essentiellement de notes de tête et de notes de cœur.

3. Système selon l'une quelconque des revendications précédentes, **caractérisé en ce que** pour retirer totalement le mélange, le corps (5, 15, 25, 35) peut se chauffer au moyen d'un dispositif de chauffage, la température de chauffe se situant dans la fourchette comprise entre 200 °C et 1000 °C.

4. Système selon la revendication 1, **caractérisé en ce que** la matière céramique du corps (5, 15, 25, 35) comporte une céramique silicatée.

5. Système selon l'une quelconque des revendications 1 et 4, **caractérisé en ce que** le corps (5, 15, 25, 35) comporte en surface une structure en relief, dotée d'un creux et / ou d'une protubérance, l'échancrure présentant de préférence une profondeur de jusqu'à 1,5 cm.

6. Système selon l'une quelconque des revendications 1 à 3, comportant additionnellement un distributeur pourvu d'un boîtier (1, 11, 21), destiné à y placer le corps (5, 15, 25, 35), le boîtier étant conçu à la manière d'une boite et le corps (5, 15, 25, 35) se plaçant dans le boîtier de sorte qu'au moins lorsque le boîtier est ouvert, le mélange puisse s'évaporer et s'échapper dans l'espace environnant.

7. Procédé, destiné à traiter le système selon l'une quelconque des revendications 1 à 3, **caractérisé en ce qu'**après l'utilisation, l'on recuit le corps (5, 15, 25, 35) poreux dans un dispositif de chauffage à une température comprise entre 200 °C et 1000 °C, de préférence entre 200 °C et 650 °C, après le recuit, le corps (5, 15, 25, 35) étant rempli une fois encore avec un mélange par exemple, de manière à ce que celui-ci soit reçu dans le corps (5, 15, 25, 35).
